# EUROPÄISCHE PATENTANMELDUNG

(11) **EP 4 473 831 A1**
(43) Veröffentlichungstag der Anmeldung: **11.12.2024**
(21) Anmeldenummer: 24157028.2
(22) Anmeldetag: 12.02.2024
(51) Int. Cl.: A01K 67/033

(54) **HÄNGEFÖRDER-AUFZUCHTVORRICHTUNG, HÄNGEFÖRDERANLAGE UND INSEKTENAUFZUCHTFARM**

(30) Priorität: 05.06.2023 DE 102023114721
(71) Anmelder: SSI Schäfer Automation GmbH (AT), 8051 Graz (AT)
(72) Erfinder: Amon, Benjamin, 8054 Graz (AT); Hintz, Andreas, 8193 Eglisau (CH)
(74) Vertreter: Witte, Weller & Partner Patentanwälte mbB

(57) **Zusammenfassung**

Es wird offenbart eine Hängeförder-Aufzuchtvorrichtung (10), aufweisend: eine Hängeeinrichtung (12) zur Kopplung an einen Hängeförderer (14); und zumindest eine Aufnahmeeinrichtung (16), die an der Hängeeinrichtung um eine horizontale erste Schwenkachse (18) schwenkbar gelagert ist, wobei die Aufnahmeeinrichtung zwischen einer ersten Stellung, in der die Aufnahmeeinrichtung im Wesentlichen vertikal ausgerichtet ist, und einer von der ersten Stellung verschiedenen zweiten Stellung um die erste Schwenkachse verschwenkbar ist und eine Mehrzahl von in der ersten Stellung übereinander angeordneten Kammern (20) zur Aufnahme von Insekten aufweist

## Beschreibung

Die vorliegende Erfindung betrifft eine Hängeförder-Aufzuchtvorrichtung.

Ferner betrifft die vorliegende Erfindung eine Hängeförderanlage sowie eine Insektenaufzuchtfarm mit einer derartigen Hängeförder-Aufzuchtvorrichtung.

Schließlich betrifft die vorliegende Erfindung ein Verfahren zum Betreiben einer derartigen Hängeförder-Aufzuchtvorrichtung.

Die globale Bevölkerung wächst stetig und mit ihr steigt auch der Bedarf an Nahrung und Proteinen. Gleichzeitig sind Ressourcen wie Ackerland, Wasser und Energie begrenzt, was dazu führt, dass traditionelle Tierhaltungssysteme nicht nachhaltig sind. Die industrielle Insektenaufzucht bietet eine vielversprechende Alternative, um diesen Bedarf zu decken, da Insekten sich durch eine hohe Proteinbiomasse auszeichnen und deutlich weniger Ressourcen benötigen als z.B. konventionelle Nutztiere.

Insekten werden in der Regel in sogenannten Insektenfarmen bzw. Insektenaufzuchtfarmen gehalten. Eine Insektenaufzuchtfarm ist ein Ort, an dem Insekten unter kontrollierten Bedingungen gezüchtet werden. Eine typische Insektenaufzuchtfarm weist mehrere Verarbeitungsstationen auf, die durchlaufen werden, um beispielsweise sicherzustellen, dass die Insekten in jeder Phase ihres Lebenszyklus optimal wachsen und gedeihen können.

Eine derartige Insektenaufzuchtfarm mit mehreren Verarbeitungsstationen ist beispielsweise aus US 2018/0070566 A1 bekannt. Als Verarbeitungsstationen beschreibt die US 2018/0070566 A1 beispielsweise eine Fütterungsstation, eine Wasserversorgungsstation, eine Aussortierungsstation zur Trennung von Insekten in unterschiedlichen Entwicklungsstadien (z.B. Eier, Larven, Puppen und adulte Insekten), eine Trennstation zur Trennung von Insekten und unverbrauchtem Substrat, eine Beladestation zum Beladen von Insekten in dafür vorgesehene Aufzuchtbehälter sowie eine Reinigungsstation zur Reinigung der Behälter.

Um die Insekten im Rahmen der Insektenaufzucht zu verarbeiten, müssen diese den verschiedenen Verarbeitungsstationen zugeführt werden. Dies kann manuell erfolgen, indem die in Aufzuchtbehältern eingelagerten Insekten den Verarbeitungsstationen händisch zugeführt werden. Alternativ könnte der Transport der Insekten mittels Förderanlagen erfolgen. Aus dem Stand der Technik sind verschiedene Förderanlagen zum Fördern von Fördergütern bekannt. Förderanlagen sind Maschinen und Anlagen, die zum Fördern von Fördergütern verwendet werden. Sie lassen sich nach der Art des Förderguts unterteilen in Förderer für Stückgut und Förderer für Schüttgut. Andere Einteilungen unterscheiden Stetig- und Unstetigförderer oder Förderer für Stückgut nach baulichen Gesichtspunkten.

Im Bereich der Insektenaufzucht kommen bislang hauptsächlich Bodenförderer, insbesondere Gurtförderer oder Rollenförderer, zum Einsatz, um Insekten in einer Aufzuchtfarm von einer Verarbeitungsstation zur nächsten zu transportieren. Zu diesem Zweck werden die Insekten regelmäßig in Aufzuchtbehältern bzw. Trays bereitgestellt, die wiederum vereinzelt und liegend auf dem Fördergutträger (z.B. auf Rollen oder auf einem Förderband) transportiert werden. Ein derartiger Bodenförderer zur Beförderung von Insekten ist beispielsweise aus der voranstehend erwähnten US 2018/0070566 A1, aus WO 2014/171829 A1 und aus US 10 362 772 B2 bekannt.

Bodenförderer benötigen in der Regel viel Platz, da für ihren Betrieb zusätzliche Stellflächen und Ressourcen am Boden benötigt werden. Dies wirkt sich nachteilig auf die Raumausnutzung einer Aufzuchtfarm aus. Ferner ist die Zufuhr von Aufzuchtbehältern auf dem Fördergutträger und die Entnahme der Aufzuchtbehälter von den Fördergutträgern komplex und aufwendig, da hierfür spezielle Handhabungsroboter benötigt werden. Die manuelle Zufuhr oder Entnahme der Aufzuchtbehälter geht wiederum zu Lasten des Automatisierungsgrades, der insbesondere in der industriellen Insektenaufzucht von Bedeutung ist.

Darüber hinaus besteht bei Bodenförderern im Zusammenhang mit der Insektenaufzucht ein Kontaminationsrisiko für die Insekten, wenn z. B. Aufzuchtbehälter während des Transports von den Bodenförderern fallen oder auf der Auflagefläche des Bodenförderers umkippen. Auch der Reinigungsaufwand ist bei Bodenförderern nicht unerheblich, da es bei der Verwendung von Bodenförderern schwierig ist, diese zwischen einzelnen Transportzyklen zu reinigen, insbesondere wenn die Bänder von klebrigen Rückständen und Insektenresten befreit werden müssen.

Schließlich ist die Verarbeitung der Aufzuchtbehälter in den jeweiligen Verarbeitungsstationen mittels herkömmlicher Bodenförderer nur begrenzt effizient. Hierbei ist einerseits zu nennen, dass die Aufzuchtbehälter individuell transportiert und gehandhabt werden. So wird beispielsweise in einer Fütterungsstation oder in einer Beladestation, in der die Insekten den Aufzuchtbehältern zugeführt werden, jeder Aufzuchtbehälter einzeln und nacheinander verarbeitet (siehe zum Beispiel US 10 362 772 B2). Ein vereinzelter Transport und eine vereinzelte Handhabung der Aufzuchtbehälter können sich jedoch ungünstig auf den Betrieb der Insektenaufzuchtfarm auswirken, da diese mit einem erheblichen Zeitaufwand einhergehen.

Auch die Lagerung der Aufzuchtbehälter gestaltet sich problematisch. Die Lagerung in herkömmlichen Aufzuchtstationen erfolgt dergestalt, dass die einzelnen Aufzuchtbehälter auf Paletten gestapelt, einzeln gestapelt oder einzeln in Regalen untergebracht sind. Da die Lagerung regelmäßig auf dem Boden, insbesondere in dafür vorgesehenen Lagerräumen der Aufzuchtstation erfolgt, besteht auch bei der Lagerung Optimierungsbedarf, insbesondere hinsichtlich der Raumausnutzung.

Aus der EP 4 062 757 A1 ist ein Verfahren zur Züchtung von adulten Insekten bekannt.

Es ist daher eine Aufgabe der vorliegenden Erfindung, eine verbesserte Insektenaufzucht zu ermöglichen. Insbesondere ist es eine Aufgabe der vorliegenden Erfindung, eine verbesserte Lagerung, einen verbesserten Transport und/oder eine verbesserte Verarbeitung von Insekten zu ermöglichen.

Gemäß einem ersten Aspekt wird eine Hängeförder-Aufzuchtvorrichtung bereitgestellt, die eine Hängeeinrichtung zur Kopplung an einen Hängeförderer und zumindest eine Aufnahmeeinrichtung, die an der Hängeeinrichtung um eine horizontale erste Schwenkachse schwenkbar gelagert ist, gelöst, wobei die Aufnahmeeinrichtung zwischen einer ersten Stellung, in der die Aufnahmeeinrichtung im Wesentlichen vertikal ausgerichtet ist, und einer von der ersten Stellung verschiedenen zweiten Stellung um die erste Schwenkachse verschwenkbar ist und eine Mehrzahl von in der ersten Stellung übereinander angeordneten Kammern zur Aufnahme von Insekten aufweist.

Hängeförderer sind aus dem Stand der Technik bekannt und dienen der Beförderung von Gütern entlang einer Führungsschiene stromabwärts in einer Förderrichtung. Derartige Hängeförderer weisen in der Regel eine Führungsschiene, angetriebene Zugmittel sowie einen Adapter auf, der entlang der Führungsschiene mittels der angetriebenen Zugmittel transportiert wird. Unter einer "Kopplung an einen Hängeförderer" wird demnach vorzugsweise eine Kopplung an eine Führungsschiene, ein Zugmittel oder einen Adapter, insbesondere an einen Rollenadapter, verstanden. Erfindungsgemäß ist zu diesem Zweck eine Hängeeinrichtung vorgesehen, die im an den Hängeförderer gekoppelten Zustand unterhalb der Führungsschiene hängt. Die Hängeeinrichtung ist im an den Hängeförderer gekoppelten Zustand vorzugsweise vertikal, d.h. mit dem Schwerefeld der Erde übereinstimmend, ausgerichtet. Es versteht sich, dass Teile der Hängeeinrichtung einen (kleinen) Winkel mit der Vertikalen bilden können. Auch derartige Ausrichtungen werden als von dem Begriff "vertikal ausgerichtet" umfasst angesehen.

Die Hängeeinrichtung kann beispielsweise als, insbesondere formstabiler bzw. starrer, Hängerahmen ausgebildet sein. An der Hängeeinrichtung ist eine Aufnahmeeinrichtung schwenkbar um eine horizontale erste Schwenkachse gelagert. Die erste Schwenkachse steht vorzugsweise im Wesentlichen senkrecht auf dem, insbesondere zum Erdmittelpunkt gerichteten, Schwerkraftvektor.

Die Aufnahmeeinrichtung weist eine Mehrzahl (d.h. mindestens zwei) Kammern zur Aufnahme von Insekten auf, die in einer ersten Stellung, in der die Aufnahmeeinrichtung im Wesentlichen vertikal ausgerichtet ist, übereinander angeordnet sind. Unter "Kammer" wird vorliegend ein zumindest teilweise geschlossener Raum verstanden, in dem die Insekten anordenbar sind. Die Kammern können beispielsweise durch in bzw. an der Aufnahmeeinrichtung aufgenommene Aufzuchtbehälter ausgebildet sein. Alternativ können die Kammern beispielsweise durch "Zellen" einer regalartig ausgebildeten Aufnahmeeinrichtung ausgebildet sein. Die Kammern können mit Mitteln ausgestattet sein, um eine kontrollierte Umgebung für die Insektenaufzucht zu schaffen. Beispielsweise können die Kammern mit speziellen Einrichtungen zur Futter- und Wasserversorgung ausgestattet sein und/oder eine bestimmte Temperatur- und/oder Feuchtigkeitsregulierung aufweisen. Vorzugsweise sind die Kammern dazu ausgebildet, die Insekten in jeder Stellung, insbesondere in der ersten Stellung und der zweiten Stellung, sicher bzw. unverlierbar aufzunehmen.

Die erste Stellung kann - bezogen auf einen Zustand, in dem die Hängeeinrichtung an den Hängeförderer gekoppelt ist - einer raumsparenden Lager- und/oder Transportstellung entsprechen. In dieser Stellung kann die Hängeförder-Aufzuchtvorrichtung platzsparend betrieben werden. Die zweite Stellung entspricht vorzugsweise einer Verarbeitungsstellung zur Verarbeitung der Insekten in einem Zustand, in dem die Hängeeinrichtung an den Hängeförderer gekoppelt ist. Dies schließt jedoch nicht aus, dass die Insekten in der ersten Stellung verarbeitet werden können.

In der zweiten Stellung ist die Aufnahmeeinrichtung vorzugsweise horizontal ausgerichtet. Die zweite Stellung der Aufnahmeeinrichtung ist jedoch nicht auf eine horizontale Ausrichtung beschränkt. Vielmehr kommen auch beliebige Ausrichtungen der Aufnahmeeinrichtung zwischen einer horizontalen und einer vertikalen Ausrichtung in Betracht. Die Lagerung der Aufnahmeeinrichtung an der Hängeeinrichtung kann derart ausgebildet sein, dass die Aufnahmeeinrichtung in der zweiten Stellung eine stabile Schwenkstellung einnimmt. Dies kann beispielsweise durch eine stufenweise Verstellbarkeit der Aufnahmeeinrichtung relativ zu der Hängeeinrichtung realisiert werden.

Das Verschwenken der Aufnahmeeinrichtung zwischen der ersten Stellung und der zweiten Stellung kann manuell bzw. händisch oder automatisiert erfolgen. Beispielsweise kann die Hängeförder-Aufzuchtvorrichtung eine oder mehrere Antriebsvorrichtungen aufweisen, die dazu ausgebildet sind, die Aufnahmeeinrichtung relativ zur Hängeeinrichtung um die horizontale Achse zu verschwenken. Denkbar ist auch, die Aufnahmeeinrichtung während eines fortgesetzten Transports in der Förderrichtung einer Hängeförderanlage gegen eine unterhalb einer Führungsschiene angeordnete Auslenkeinrichtung zu verfahren, um die Aufnahmeeinrichtung aus der ersten Stellung in die zweite Stellung zu drehen.

Der Begriff "Insekten" umfasst vorliegend sämtliche Entwicklungsstadien bzw. Erscheinungsformen eines Insekts, von der Eiablage über die Larven- und Puppenphase bis hin zur adulten Form, also dem ausgewachsenen Insekt. Die Aufzucht von Insekten erfolgt in der Regel unter Verwendung von Substraten und einer geeigneten Nahrung. Je nach Art der Insekten können verschiedene Substrate wie beispielsweise Weizenkleie, Sägemehl oder Gemüsereste verwendet werden. Die Insekten werden auf diesen Substraten gehalten und mit einer geeigneten Nahrung gefüttert, um ihr Wachstum und ihre Entwicklung zu fördern. Vorzugsweise sind die Kammern dazu ausgebildet, neben Insekten auch ein Substrat und/oder geeignete Nahrung aufzunehmen.

Durch die erfindungsgemäße Anordnung der Kammern in Verbindung mit der Schwenkbarkeit der die Kammern aufweisenden Aufnahmeeinrichtung wird ein raumsparender Transport und/oder eine raumsparende Lagerung der Insekten ermöglicht. Die erste Stellung der Aufnahmeeinrichtung ist besonders dann von Vorteil, wenn eine Vielzahl von Hängeförder-Aufzuchtvorrichtungen mit einem Hängeförderer gekoppelt sind, da so ein kompakter Transport der Hängeförder-Aufzuchtvorrichtungen und/oder eine kompakte Anordnung der Hängeförder-Aufzuchtvorrichtungen zu Lagerzwecken gewährleistet werden kann.

Die Schwenkbarkeit der Aufnahmeeinrichtung bietet zudem eine höhere Flexibilität hinsichtlich der Insektenverarbeitung, da die Ausrichtung bzw. Orientierung der Aufnahmeeinrichtung individuell an die Gegebenheiten einer Verarbeitungsstation anpassbar ist.

Im Gegensatz zu Bodenförderern, die die Insekten mit Hilfe von Insektenaufzuchtbehältern auf einer horizontalen Fläche transportieren, ermöglicht die erfindungsgemäße Hängeförder-Aufzuchtvorrichtung eine hängende Förderung und/oder Lagerung der Insekten, was sich ebenfalls vorteilhaft auf die Raumausnutzung, insbesondere in einer Aufzuchtfarm, auswirkt. Mit anderen Worten können die Insekten mittels der Hängeförder-Aufzuchtvorrichtung im an den Hängeförderer gekoppelten Zustand befördert und/oder hängend gelagert werden, wodurch Ressourcen und Platz gespart werden. Da aufgrund des Einsatzes von Hängefördertechnik zur Insektenbeförderung- und/oder Insektenlagerung der Boden im Wesentlichen frei von festen technischen Einrichtungen zur Beförderung und/oder Lagerung der Aufzuchtbehälter bleibt, ist dieser leichter sauber zu halten bzw. zu reinigen. Auch können manuell bediente Unstetigförderer und/oder sogenannte "Autonomous Mobile Robots" (AMRs) ungehindert verfahren.

In einer Ausgestaltung des ersten Aspekts ist die zumindest eine Aufnahmeeinrichtung in der zweiten Stellung im Wesentlichen horizontal ausgerichtet.

Eine horizontale Ausrichtung eignet sich besonders zur Verarbeitung der Insekten, insbesondere in einer Verarbeitungsstation einer Aufzuchtfarm. In dieser Stellung können die Kammern besonders einfach mit Insekten, Futter und/oder Substrat beladen werden. Eine horizontale Stellung kann sich ebenfalls als vorteilhaft für eine Bewässerung der Insekten erweisen. Auch ein Entleeren der Kammern in der zweiten Stellung ist denkbar. Es versteht sich, dass Teile der Aufnahmeeinrichtung einen (kleinen) Winkel mit der Horizontalen bilden können. Auch derartige Ausrichtungen werden als von dem Begriff "horizontal ausgerichtet" umfasst angesehen.

In einer weiteren Ausgestaltung des ersten Aspekts weist die Aufnahmeeinrichtung einen Grundkörper oder einen Rahmen mit, insbesondere einander gegenüberliegenden, Stirnseiten, einer Bodenwand, einer Deckwand, einander gegenüberliegenden Seitenwänden und zumindest einer Trennwand, die sich zwischen den Seitenwänden erstreckt und einen Innenraum des Rahmens in die Mehrzahl von Kammern unterteilt, auf.

Die Trennwand und der Boden des Grundkörpers bzw. des Rahmens fungieren in der ersten Stellung der Aufnahmeeinrichtung als "Tragböden" bzw. Auflageflächen für die Insekten und sind in dieser Stellung vorzugsweise im Wesentlichen horizontal ausgerichtet. Die zumindest eine Trennwand, die sich zwischen den Seitenwänden des Grundkörpers bzw. des Rahmens erstreckt, trennt zwei (in Vertikalrichtung) benachbarte Kammern voneinander. Vorzugsweise kontaktiert die zumindest eine Trennwand, die sich zwischen den Seitenwänden erstreckt, die Seitenwände, um einen Insektenaustausch zwischen benachbarten Kammern zu vermeiden. Die zumindest eine Trennwand ist vorzugsweise als ebene Platte ausgebildet, kann jedoch auch andere Formen oder Profile annehmen. Beispielsweise kann die zumindest eine Trennwand mit Vertiefungen oder Nuten zur Aufnahme der Insekten versehen sein.

Die Seitenwände, die Bodenwand und/oder die zumindest eine Trennwand sind vorzugsweise jeweils geschlossen ausgebildet. Alternativ kann vorgesehen sein, dass die Seitenwände, die Bodenwand und/oder die zumindest eine Trennwand jeweils zumindest teilweise geöffnet oder öffenbar ausgebildet sind. Beispielsweise können Öffnungen und/oder Kanäle in den Seitenwänden, der Bodenwand und/oder der zumindest einen Trennwand vorgesehen sein, die zur Belüftung und/oder zur Bewässerung dienen können.

Die Stirnseiten des Rahmens, die auch als "Vorderseite" und als "Rückseite" bezeichnet werden können, können zumindest teilweise verschließbar oder geschlossen ausgebildet sein. Die Begriffe "Rückseite" und "Vorderseite" bzw. "rückseitig" und "vorderseitig" dienen lediglich der Unterscheidung der Stirnseiten des Grundkörpers bzw. des Rahmens und implizieren nicht zwingend eine Orientierung der Aufnahmeeinrichtung, wenn die Hängeförder-Aufzuchtvorrichtung an den Hängeförderer gekoppelt ist. Es versteht sich von selbst, dass der Grundkörper bzw. der Rahmen derart ausgebildet ist, dass eine, insbesondere wahlweise, Zugänglichkeit der Kammern sichergestellt ist, beispielsweise um die Kammern zu beladen oder zu entladen. Beispielsweise kann vorgesehen sein, dass wenigstens einer der Stirnseiten des Grundkörpers bzw. des Rahmens die Kammern stirnseitig zumindest teilweise verschließt, um ein Herausfallen der Insekten aus den Kammern in der ersten und/oder zweiten Stellung zu vermeiden. Mit anderen Worten kann zumindest eine der beiden Stirnseiten des Rahmens als Begrenzungselement ausgebildet sein, um ein stirnseitiges Herausfallen der Insekten, insbesondere in der zweiten Stellung, zu vermeiden. Wird die Aufnahmeeinrichtung beispielsweise aus der Vertikalen in die Horizontale verschwenkt, so kann die entsprechende Stirnseite in dieser Stellung als Tragboden bzw. Auflagefläche für die Insekten dienen.

Der Grundkörper bzw. der Rahmen ist vorzugweise zumindest teilweise formstabil bzw. starr ausgebildet. Insbesondere kann vorgesehen sein, dass die Seitenwände formstabil ausgebildet sind. Alternativ oder ergänzend kann zumindest eine der Stirnseiten formstabil bzw. starr ausgebildet sein.

Die Aufnahmeeinrichtung kann in dieser bevorzugten Ausgestaltung beispielsweise als ein an der Hängeeinrichtung drehbar gelagerter Insektenaufzuchtbehälter bzw. Tray ausgebildet sein. Der Boden des Aufzuchtbehälters entspricht dabei einer der voranstehend beschriebenen Stirnseiten des Grundkörpers, währen die Bodenwand, die Kopfwand und die Seitenwände den aufragenden Wänden des Insektenaufzuchtbehälters entsprechen. Die zumindest eine Trennwand unterteilt den Innenraum des so gebildeten Aufzuchtbehälters in die verschiedenen Kammern. Die übrige Stirnseite kann beispielsweise als "Deckel" des Aufzuchtbehälters ausgebildet sein.

In einer weiteren Ausgestaltung des ersten Aspekts erstrecken sich die Kammern zwischen den Stirnseiten des Grundkörpers bzw. des Rahmens.

Mit anderen Worten erstrecken sich die die Kammer umschließenden Wandelemente, d.h. der als Trennwand oder als Rahmenboden ausgebildete Tragboden, die Seitenwände und das als Trennwand oder als Deckwand ausgebildete Deckelement einer jeder Kammer zwischen den Stirnseiten des Grundkörpers bzw. des Rahmens.

In einer weiteren Ausgestaltung des ersten Aspekts sind die Kammern zu zumindest einer der beiden Stirnseiten hin geschlossen oder verschließbar.

Beispielsweise kann eine der Stirnseiten der Kammern offen ausgebildet sein, während die andere (gegenüberliegende) Stirnseite geschlossen oder verschließbar ausgebildet ist. Alternativ können beide Stirnseiten der Kammern verschließbar ausgebildet sein. "Verschließbar" ist vorliegend dahingehend zu verstehen, dass die jeweilige Kammer zu der entsprechenden Stirnseite hin nicht irreversibel geschlossen ist, sondern geöffnet und geschlossen werden kann, so dass durch eine entsprechende stirnseitige Öffnung der Kammer die Insekten aus der Kammer entnommen werden oder dieser zugeführt werden können.

In einer weiteren Ausgestaltung des ersten Aspekts ist zumindest eine der Stirnseiten des Grundkörpers bzw. des Rahmens als Wandung ausgebildet, wobei die Wandung die Mehrzahl von Kammern stirnseitig verschließt.

Die Wandung kann geschlossen ausgebildet sein oder zumindest teilweise mit Öffnungen versehen sein. Die Wandung weist vorzugsweise eine Vielzahl von Öffnungen auf. Beispielsweise kann die Wandung gelocht, siebförmig oder perforiert ausgebildet sein. Alternativ kann die Wandung netzartig, gitterartig oder gewebeartig ausgebildet sein. Die Öffnungen der Wandung sind vorzugsweise klein genug, um ein Herausfallen der in den Kammern aufgenommenen Insekten, insbesondere in der zweiten Stellung, zu verhindern, jedoch groß genug, um eine ausreichende Belüftung und/oder Reinigung zu ermöglichen. Vorzugsweise beträgt der mittlere Durchmesser der Öffnungen 0,05 bis 6 mm, insbesondere 0,1 bis 5 mm. Die Wandung kann starr oder biegeweich bzw. biegsam ausgebildet sein. Die Wandung ist vorzugsweise lösbar an zumindest einer der Stirnseiten des Grundkörpers bzw. des Rahmens angebracht.

Diese bevorzugte Ausgestaltung erweist sich als vorteilhaft in Bezug auf ein automatisiertes Öffnen und Verschließen und in Bezug auf ein regelmäßiges Reinigen. Durch die bevorzugten Öffnungen können die Insekten mit ausreichend Sauerstoff versorgt werden. Darüber hinaus erleichtern die Öffnungen eine Bewässerung der mit Insekten beladenen Kammern und damit eine Befeuchtung der Insekten bzw. des Substrats.

In einer besonders bevorzugten Ausgestaltung ist die Wandung als Gewebe, insbesondere als Gaze, ausgebildet, das die Kammern stirnseitig bespannt. Das Gewebe kann biegeweich bzw. biegsam oder starr ausgebildet sein.

Mit anderen Worten sind die Kammern stirnseitig mit einem netzartigen oder gitterartigen Gewebe versehen, um ein Herausfallen der in den Kammern aufgenommenen Insekten, insbesondere in der zweiten Stellung, zu verhindern. Die Wahl der Porengröße des Gewebes hängt von der Größe der zu transportierenden Insekten ab. Im Allgemeinen sollten die Poren des Gewebes klein genug sein, um zu verhindern, dass die Insekten durch das Gewebe fallen, aber groß genug, um eine ausreichende Belüftung zu ermöglichen. Vorzugsweise beträgt die (durchschnittliche) Porengröße des Gewebes 0,05 bis 6 mm, insbesondere 0,1 bis 5 mm. Das Gewebe ist vorzugsweise lösbar an zumindest einer der Stirnseiten des Grundkörpers bzw. des Rahmens angebracht. Beispielsweise kann das Gewebe durch miteinander verbundene Streben aufgespannt sein, die lösbar mit dem Grundkörper bzw. mit dem Rahmen koppelbar sind. Alternativ kann das Gewebe beispielsweise mittels eines Klettverschlusses an diesem angebracht sein.

Ein Vorteil dieser bevorzugten Ausgestaltung ist, dass die Insekten mit ausreichend Sauerstoff versorgt werden können. Darüber hinaus erleichtern die Poren des Gewebes eine Bewässerung der mit Insekten beladenen Kammern und damit eine Befeuchtung der Insekten bzw. des Substrats.

In einer weiteren Ausgestaltung des ersten Aspekts weist die zumindest eine Aufnahmeeinrichtung eine Mehrzahl von Behältern zur Aufnahme der Insekten und eine, insbesondere zumindest teilweise formstabile, Haltestruktur zum Halten der Mehrzahl von Behältern auf, wobei die Behälter in der ersten Stellung übereinander angeordnet sind, und wobei jeder Behälter der Mehrzahl von Behältern eine Kammer der Mehrzahl von Kammern ausbildet.

Demnach bilden die in der ersten Stellung der Aufnahmeeinrichtung übereinander angeordneten Behälter die Kammern aus. Die Haltestruktur ist zum Halten der Behälter ausgebildet. Beispielsweise können Behälter an der Haltestruktur, insbesondere lösbar, befestigt sein. Die Behälter können jedoch auch Teil der Haltestruktur sein. Beispielsweise können die Behälter einstückig bzw. integral mit der Haltestruktur ausgebildet sein. Optional kann vorgesehen sein, dass die Behälter jeweils drehbar an der Haltestruktur gelagert sind. Bei den Behältern kann es sich beispielsweise um Aufzuchtbehälter handeln. Die zur Insektenaufnahme ausgebildeten Behälter sind vorzugsweise formstabil bzw. starr ausgebildet. Der Begriff "formstabil" bezieht sich auf die Eigenschaft des Behälters, unter Last seine Form beizubehalten, ohne dass er während der Verwendung durch äußere Kräfte oder interne Belastung verformt wird. Mit anderen Worten behält ein formstabiler Behälter seine vorgegebene Form bei. Die Behälter können aus verschiedenen Materialen hergestellt sein. Beispielsweise können die Behälter aus Kunststoff (z.B. Polypropylen (PP), Polyethylen (PE) oder Polycarbonat (PC)) hergestellt sein, da Kunststoffe langlebig, leicht zu reinigen und sterilisierbar sind. Alternativ kommen Materialien wie Metall oder Glas in Betracht, die in der Regel schwerer und weniger flexibel als Kunststoff sind.

Die Behälter und/oder die Haltestruktur können auch ganz oder teilweise aus biologisch abbaubaren Materialien hergestellt sein um den Insekten in verschiedenen Entwicklungsstadien, zumindest teilweise, als Nahrung zu dienen und die Insektenentwicklung positiv zu beeinflussen, indem ihnen ein natürlicherer Lebensraum zur Verfügung gestellt wird. Neben der Massenherstellung solcher Vorrichtungen ist es möglich, mittels 3D-Druckverfahren regelmäßige und/oder zum Teil zufällige Strukturen herzustellen, die auf besondere, spezifische Insektenvorlieben Rücksicht nehmen und damit deren Aufzucht oder Vermehrung begünstigen oder erst ermöglichen und in üblichen Massenherstellverfahren nicht oder nicht einstückig herstellbar sind.

Es versteht sich, dass diese Vorrichtungen, Behälter und/oder Haltestrukturen schwierig oder gar nicht zu reinigen und daher weniger lang einsetzbar sind. Zu Erntezwecken kann es außerdem erforderlich sein, die Behälter und/oder Haltestrukturen zumindest teilweise zu zerstören, um die erntereifen Insekten zu erreichen.

"Halten" meint hier, dass die Haltestruktur die Behälter in den verschiedenen Schwenkstellungen sicher bzw. unverlierbar aufnimmt. Beispielsweise können die Behälter an der Haltestruktur, insbesondere lösbar, befestigt sein. Hierbei kommen insbesondere formschlüssige oder kraftschlüssige Verbindungen in Betracht. Beispielsweise können die Behälter mittels eines Rastmechanismus oder eines Klippmechanismus an der Haltestruktur gehalten sein. Lösbare Verbindungen ermöglichen einen einfachen Austausch oder eine einfache Reinigung der Behältnisse.

Der Vorteil dieser bevorzugten Ausgestaltung ist, dass eine kompakte Anordnung der Behälter und damit ein effizienter Betrieb der Hängeförder-Aufzuchtvorrichtung sichergestellt werden kann. Ferner ermöglicht diese bevorzugte Ausgestaltung ein Nachrüsten der Hängeförder-Aufzuchtvorrichtung mit herkömmlichen Insektenaufzuchtbehältern.

In einer weiteren Ausgestaltung des ersten Aspekts weist jeder Behälter einen Behälterboden und eine den Behälterboden zumindest teilweise, insbesondere vollständig, umgebende und von dem Behälterboden aufragende Behälterseitenwand auf, insbesondere wobei jeder Behälterboden in der ersten Stellung im Wesentlichen horizontal ausgerichtet ist.

Die Behälterseitenwand ragt in der ersten Stellung der Aufnahmeeinrichtung nach oben und sorgt dafür, dass die auf den Behälterböden aufliegenden Insekten nicht aus den jeweiligen Kammern herausfallen. Vorzugsweise sind die Behälterböden in der ersten Stellung im Wesentlichen horizontal ausgerichtet. Es versteht sich, dass Teile des Behälterbodens einen (kleinen) Winkel mit der Horizontalen bilden können. Auch derartige Ausrichtungen werden als von dem Begriff "horizontal ausgerichtet" umfasst angesehen.

Wird die Aufnahmeeinrichtung in die zweite Stellung verschwenkt, kann es je nach Neigungswinkel passieren, dass die Insekten schwerkraftbedingt von dem Behälterboden allmählich auf die Behälterseitenwand verlagert werden. Insbesondere bei nach oben hin geöffneten bzw. unverschlossenen Behältern droht dann ein Herausfallen der Insekten. Um dies zu vermeiden, kann die von dem Behälterboden aufragende Behälterseitenwand zumindest abschnittsweise zu einem Behälterinneren hin gekrümmt oder geneigt ausgebildet sein. Durch die Krümmung oder Neigung der Behälterwand kann ein Herausfallen der Insekten aus dem Behälter während einer Schwenkbewegung der Haltestruktur verhindert werden. Darüber hinaus kann durch diese Ausgestaltung des Behälters auf einen Behälterdeckel verzichtet werden, was für die Aufzucht der Insekten von Vorteil ist (z.B. im Hinblick auf eine verbesserte Sauerstoffversorgung).

In einer weiteren Ausgestaltung des ersten Aspekts weist die Haltestruktur eine Rückwand und zwei von der Rückwand nach vorn abstehende, insbesondere formstabile, Seitenwände, zwischen denen die Mehrzahl von Behältern gehalten ist, auf, wobei die sich zwischen den Seitenwänden erstreckenden Behälter die Rückwand kontaktieren, insbesondere wobei die Seitenwände an der Hängeeinrichtung um die horizontale erste Schwenkachse schwenkbar gelagert sind.

Da die Behälter die Rückwand kontaktieren bzw. an dieser anliegen und sich (entlang einer Breitseite der Rückwand) zwischen zwei relativ zu der Rückwand nach vorn ragenden Seitenwänden erstrecken, dient die Rückwand als Begrenzungsfläche, um bspw. aus den Behältern in der zweiten Stellung herausfallende Insekten "aufzufangen". Die Rückwand kann bspw. in der ersten Stellung im Wesentlichen vertikal ausgerichtet sein. Die Rückwand kann formstabil ausgebildet oder aus einem flexiblen Material hergestellt sein.

In einer weiteren Ausgestaltung des ersten Aspekts ist die Rückwand als Gewebe, insbesondere als Gaze, ausgebildet, das die Behälter überspannt.

Die Wahl der Porengröße des Gewebes hängt von der Größe der zu transportierenden Insekten ab. Im Allgemeinen sollten die Poren des Gewebes klein genug sein, um zu verhindern, dass die Insekten durch das Gewebe fallen. Vorzugsweise beträgt die (durchschnittliche) Porengröße des Gewebes 0,05 bis 6 mm, insbesondere 0,1 bis 5 mm. Das Gewebe ist vorzugsweise lösbar an der Haltestruktur (z.B. an den Seitenwänden) angebracht, um die Behälter zu überspannen. Beispielsweise kann das Gewebe durch miteinander verbundene Streben aufgespannt sein, die lösbar mit der Haltestruktur koppelbar sind.

Die Behälter sind vorzugsweise derart an der Haltestruktur gehalten, dass das aufgespannte Gewebe die in der ersten Stellung übereinander angeordneten Behälter rückseitig kontaktiert. Dieser Kontakt bleibt durch die Oberflächenspannung des aufgespannten Gewebes in den verschiedenen Schwenkstellungen erhalten und verhindert ein Herausfallen der Insekten aus den Kammern.

In einer weiteren Ausgestaltung des ersten Aspekts ist der zumindest eine Behälter drehbar um eine zweite Schwenkachse an der Haltestruktur gelagert.

Mit anderen Worten sind die Behälter relativ zu der Haltestruktur drehbar. Ein Vorteil dieser Ausgestaltung ist, dass die Hängeförder-Aufzuchtvorrichtung aufgrund des zusätzlichen Freiheitsgrades der Behälter flexibler ist. So kann beispielsweise eine Orientierung der Behältnisse relativ zu der Haltestruktur (individuell) eingestellt werden.

In einer weiteren Ausgestaltung des ersten Aspekts verläuft die zweite Schwenkachse parallel zu der ersten Schwenkachse.

Ein Vorteil dieser Ausgestaltung ist, dass die Behälter kompakt anordenbar und deren Orientierung einfach eingestellt werden kann.

In einer weiteren Ausgestaltung des ersten Aspekts ist der zumindest eine Behälter derart frei drehbar an der Haltestruktur gelagert, dass der zumindest eine Behälter zumindest in der ersten Stellung und in der zweiten Stellung, insbesondere zwischen der ersten Stellung und der zweiten Stellung, orientierungsstabil ist

"Orientierungsstabil" meint hier, dass eine Orientierung der Behälter in der ersten Stellung im Wesentlichen einer Orientierung der Behälter in der zweiten Stellung entspricht. Durch die frei drehbare Lagerung der Behälter kann somit eine für die Insektenaufzucht optimale Orientierung der Behälter unabhängig von der Schwenkstellung der Aufnahmeeinrichtung aufrecht erhalten werden. Dadurch kann sichergestellt werden, dass die in den Kammern aufgenommenen Insekten ihre Lage beibehalten, d.h. während einer Drehung der Aufnahmeeinrichtung nicht umherfliegen. Dies wirkt sich vorteilhaft auf die Insektenaufzucht bzw. das Insektenwachstum aus, da die Insekten weniger Stress ausgesetzt sind.

Beispielsweise können die Tragböden der Behälter zumindest in der ersten Stellung und in der zweiten Stellung im Wesentlichen horizontal ausgerichtet sein. Eine im Wesentlichen horizontale Ausrichtung der Behälterböden eignet sich besonders für die Insektenaufzucht.

Vorzugsweise verläuft die zweite Schwenkachse nicht durch einen Schwerpunkt eines jeden Behälters. Dadurch kann sichergestellt werden, dass sich die Behälter immer wieder in ihre Ausgangslage zurückkehren.

In einer weiteren Ausgestaltung des ersten Aspekts ist die Hängeeinrichtung in der ersten Stellung und in der zweiten Stellung im Wesentlichen vertikal ausgerichtet.

Mit anderen Worten ist die Hängeeinrichtung, die das Verbindungsglied zwischen dem Hängeförderer und der verschwenkbaren Aufnahmeeinrichtung darstellt, unabhängig von der Schwenkbewegung der Aufnahmeeinrichtung im Wesentlichen vertikal ausgerichtet.

In einer weiteren Ausgestaltung des ersten Aspekts kann vorgesehen sein, dass die Hängeeinrichtung, insbesondere im an den Hängeförderer gekoppelten Zustand, um eine vertikale Achse drehbar ausgebildet ist.

Auf diese Weise kann eine Orientierung der Hängeförder-Aufzuchtvorrichtung um die vertikale Achse nach Bedarf geändert werden. Dies kann insbesondere für den Transport und/oder die Lagerung der Hängeförder-Aufzuchtvorrichtung vorteilhaft sein.

Gemäß einem zweiten Aspekt wird eine Hängeförderanlage zur Beförderung von Insekten bereitgestellt, mit zumindest einer Hängeförder-Aufzuchtvorrichtung nach dem zweiten Aspekt, und einem Hängeförderer zur Beförderung der zumindest einen Hängeförder-Aufzuchtvorrichtung entlang einer Führungsschiene stromabwärts in einer Förderrichtung.

Die Hängeförderanlage kann beispielsweise in einer Insektenaufzuchtfarm eingesetzt werden, um die Insekten hängend zwischen verschiedenen Verarbeitungsstationen zu transportieren. Neben dem Insektentransport eignet sich die Hängeförderanlage insbesondere auch zur Insektenlagerung. Eine bodenseitige Lagerung bzw. ein bodenseitiger Transport der Insekten kann somit vermieden werden, was sich wiederum positiv auf die Raumausnutzung auswirkt.

Gemäß einem dritten Aspekt wird eine Insektenaufzuchtfarm bereitgestellt, aufweisend: zumindest eine Verarbeitungsstation zur Verarbeitung der Insekten; und eine Hängeförderanlage mit zumindest einer Hängeförderer-Aufzuchtvorrichtung nach dem ersten Aspekt und einem Hängeförderer zur Beförderung der zumindest einen Hängeförderer-Aufzuchtvorrichtung entlang einer Führungsschiene zu der zumindest einen Verarbeitungsstation und/oder von der zumindest einen Verarbeitungsstation weg.

Vorzugsweise weist die Insektenaufzuchtfarm eine Mehrzahl von Verarbeitungsstationen auf und der Hängeförderer ist zur Beförderung der zumindest einen Hängeförder-Aufzuchtvorrichtung entlang der Führungsschiene zwischen der Mehrzahl von Verarbeitungsstationen ausgebildet.

Unter "Verarbeitungsstation" wird ein Bereich oder ein Raum einer Insektenaufzuchtfarm verstanden, in dem die Insekten verarbeitet (z.B. sortiert, gereinigt, gefüttert, aufbereitet, etc.) werden. Eine Verarbeitungsstation kann beispielsweise eine Fütterungsstation, eine Wasserversorgungsstation, eine Sortierstation, eine Beladestation zum Einbringen der Insekten in die Kammern, eine Entladestation zum Entleeren der Kammern, eine Reinigungsstation oder dergleichen sein. Alternativ kann eine Verarbeitungsstation eine Lagerstation zur hängenden Lagerung der Insekten sein.

Gemäß einem vierten Aspekt wird ein, insbesondere automatisiertes, Verfahren zum Betreiben einer Hängeförder-Aufzuchtvorrichtung nach dem ersten Aspekt bereitgestellt, das die folgenden Schritte aufweist:
- Bereitstellen der zumindest einen an einen Hängeförderer einer Hängeförderanlage gekoppelten Hängeförder-Aufzuchtvorrichtung in einer Verarbeitungsstation einer Insektenaufzuchtfarm;
- Schwenken der Aufnahmeeinrichtung zwischen der ersten Stellung und der zweiten Stellung; und
- Verarbeiten der Insekten in der Verarbeitungsstation, während die Aufnahmeeinrichtung sich in der ersten Stellung oder in der zweiten Stellung befindet.

In der Insektenaufzucht kann es je nach Verarbeitungsstation sinnvoll sein, die Hängeförder-Aufzuchtvorrichtung in der ersten Stellung oder in der zweiten Stellung zu betreiben, um die Insekten zu verarbeiten. Beispielsweise kann sich die zweite Stellung der Aufnahmeeinrichtung besonders für ein Beladen der Kammern mit Insekten oder Entladen der Kammern eignen, da aufgrund der erfindungsgemäßen Anordnung der Kammern übereinander die Kammern in der zweiten Stellung besser zugänglich sind. Entsprechendes kann für eine Bewässerung bzw. Befeuchtung der Insekten gelten. Andererseits kann sich die Vertikalstellung beispielsweise in einer Reinigungsstation oder einer Lagerstation als vorteilhaft erweisen.

Vorzugsweise umfasst der Schritt des Verarbeitens der Insekten einen Schritt des Beladens der Mehrzahl von Kammern der zumindest einen Hängeförder-Aufzuchtvorrichtung mit den Insekten und/oder einen Schritt des Entladens der Mehrzahl von Kammern der zumindest einen Hängeförder-Aufzuchtvorrichtung, während sich die Aufnahmeeinrichtung in der zweiten Stellung befindet.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsformen der Erfindung sind in der Zeichnung dargestellt und werden in der nachfolgenden Beschreibung näher erläutert. Es zeigen:
- Fig. 1A: eine schematische Darstellung einer Ausführungsform einer Hängeförder-Aufzuchtvorrichtung;
- Fig. 1B: eine Seitenansicht der Hängeförder-Aufzuchtvorrichtung aus Fig. 1A in einer ersten Stellung;
- Fig. 1C: eine Seitenansicht der Hängeförder-Aufzuchtvorrichtung aus Fig. 1A in einer zweiten Stellung;
- Fig. 2: eine isometrische Ansicht einer bevorzugten Ausführungsform der Hängeförder-Aufzuchtvorrichtung aus Fig. 1A;
- Fig. 3: eine isometrische Ansicht einer bevorzugten Ausführungsform der Hängeförder-Aufzuchtvorrichtung aus Fig. 1A; und
- Fig. 4: eine schematische Darstellung einer Ausführungsform einer Hängeförderanlage;
- Fig. 5: eine schematische Darstellung einer Ausführungsform einer Insektenaufzuchtfarm;
- Fig. 6: ein Flussdiagramm eines Verfahrens zum Betreiben einer Hängeförder-Aufzuchtvorrichtung.

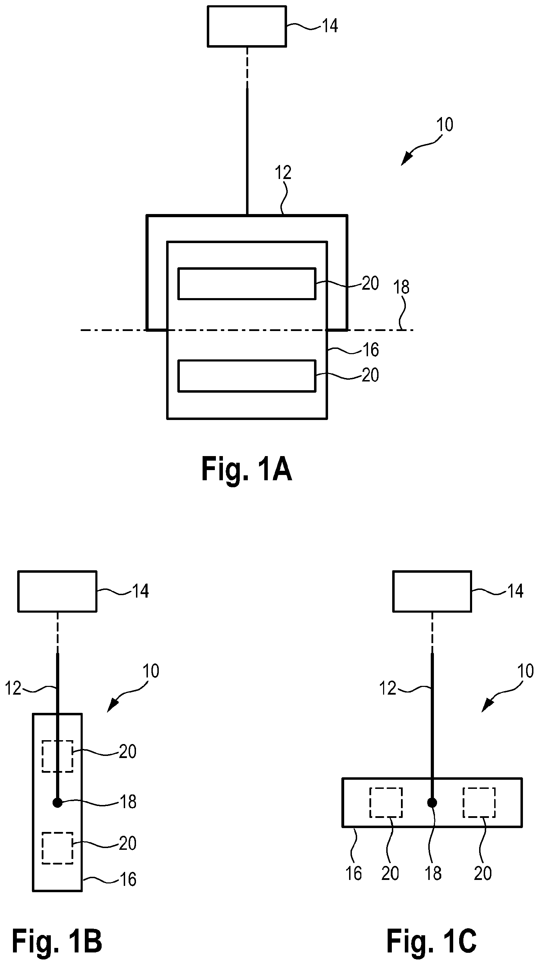

Fig. 1A zeigt eine schematische Darstellung einer ersten Ausführungsform einer Hängeförder-Transportvorrichtung 10 gemäß einem ersten Aspekt der Erfindung.

Die Hängeförder-Aufzuchtvorrichtung 10 der ersten Ausführungsform weist eine Hängeeinrichtung 12 zur Kopplung an einen Hängeförderer 14 auf. Der Hängeförderer 14 kann beispielsweise eine Führungsschiene, ein Zugmittel oder ein Rollen-Adapter sein. Des Weiteren weist die Hängeförder-Aufzuchtvorrichtung 10 eine Aufnahmeeinrichtung 16 auf, die um eine horizontale Schwenkachse 18 schwenkbar an der Hängeeinrichtung 12 gelagert ist. Die Aufnahmeeinrichtung 16 weist Kammern 20 zur Aufnahme von Insekten (z.B. Eier, Larven, Puppen und adulte Insekten) auf. In der in Fig. 1A dargestellten ersten Stellung, in der die Aufnahmeeinrichtung im Wesentlichen vertikal (d.h. im Wesentlichen mit dem Schwerefeld der Erde übereinstimmend) ausgerichtet ist, sind die Kammern 20 in einer Höhenrichtung übereinander angeordnet. Die Aufnahmeeinrichtung 16 ist zwischen der ersten Stellung und einer zweiten Stellung um die horizontale Schwenkachse 18 verschwenkbar.

Die erste Stellung und die zweite Stellung der Aufnahmeeinrichtung 16 sind exemplarisch in den Fig. 1B und 1C dargestellt. Dabei zeigt Fig. 1B eine Seitenansicht der Hängeförder-Aufzuchtvorrichtung 10 aus Fig. 1A in der ersten Stellung und Fig. 1B eine Seitenansicht der Hängeförder-Aufzuchtvorrichtung 10 aus Fig. 1A in einer zweiten Stellung. In der in Fig. 1C gezeigten Darstellung ist die Aufnahmeeinrichtung 16 in der zweiten Stellung exemplarisch im Wesentlichen horizontal (d.h. um 90 Grad gegenüber der vertikalen Ausrichtung gedreht) ausgerichtet. Eine im Wesentlichen horizontale Ausrichtung eignet sich besonders zur Verarbeitung der Insekten. Beispielsweise können die Kammern 20 in der horizontalen Stellung besonders einfach mit Insekten, Futter und/oder Substrat beladen werden. Eine horizontale Stellung kann sich ebenfalls als vorteilhaft für z.B. eine Bewässerung der Insekten und/oder ein Entleeren der Kammern erweisen.

Das Verschwenken der Aufnahmeeinrichtung 16 zwischen der in den Fig. 1A und 1B dargestellten ersten Stellung und der in der Fig. 1C dargestellten zweiten Stellung kann manuell erfolgen. Alternativ kann das Verschwenken automatisiert erfolgen. Beispielsweise kann die Hängeförder-Aufzuchtvorrichtung 10 eine oder mehrere Antriebsvorrichtungen (nicht dargestellt) aufweisen, die dazu ausgebildet sind, die Aufnahmeeinrichtung 16 relativ zu der Hängeeinrichtung 12 um die horizontale Schwenkachse 18 zu verschwenken. Denkbar ist auch, die Aufnahmeeinrichtung 16 während eines fortgesetzten Transports der Hängeförder-Aufzuchtvorrichtung 10 in einer Förderrichtung gegen eine unterhalb einer Führungsschiene 14 angeordnete Auslenkeinrichtung (nicht dargestellt) zu verfahren, um die Aufnahmeeinrichtung 16 aus der ersten Stellung in die zweite Stellung zu drehen.

Die Fig. 1A bis 1C zeigen Konfigurationen der Hängeförder-Aufzuchtvorrichtung 10 mit einer (einzigen) Aufnahmeeinrichtung 16. Die Hängeförder-Aufzuchtvorrichtung 10 kann jedoch eine Vielzahl von Aufnahmeeinrichtungen 16 aufweisen, von denen jede um eine horizontale Schwenkachse 18 an der Hängeeinrichtung 12 drehbar gelagert ist. Die Aufnahmeeinrichtungen 16 können in der ersten Stellung beispielsweise nebeneinander und/oder übereinander angeordnet sind.

Die Hängeförder-Aufzuchtvorrichtung 10 ermöglicht, insbesondere im an den Hängeförderer 14 gekoppelten Zustand, eine verbesserte Lagerung, einen verbesserten Transport und/oder eine verbesserte Verarbeitung der Insekten.

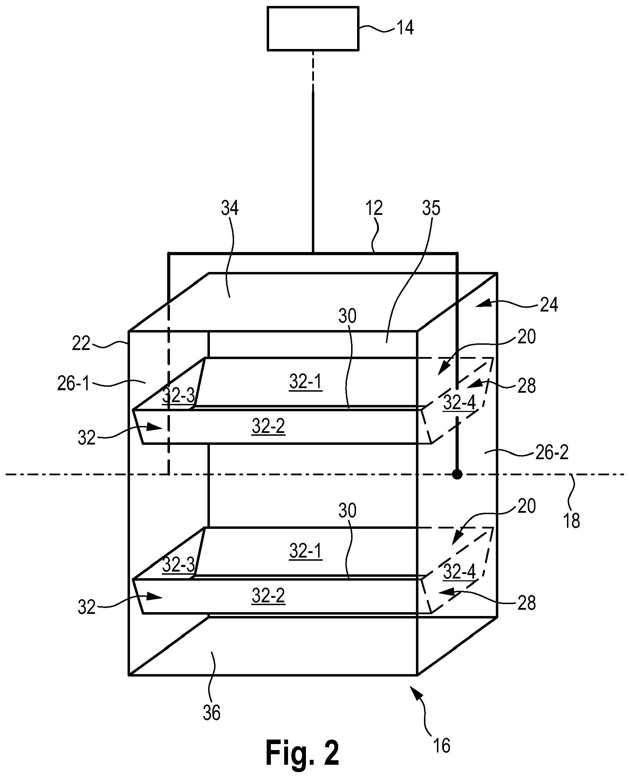

Fig. 2 zeigt eine isometrische Ansicht einer bevorzugten Ausführungsform der Hängeförder-Aufzuchtvorrichtung 10 aus Fig. 1A. Gleiche Elemente sind mit gleichen Bezugszeichen gekennzeichnet und werden nicht näher erläutert.

In der in Fig. 2 dargestellten Ausführungsform der Hängeförder-Aufzuchtvorrichtung 10 befindet sich die Aufnahmeeinrichtung 16 exemplarisch in der ersten Stellung. Die Aufnahmeeinrichtung 16 weist eine Haltestruktur 22 auf. Die Haltestruktur 22 weist eine Rückwand 24 und zwei von der Rückwand 24 nach vorn abstehende, insbesondere formstabile, Seitenwände 26-1 und 26-2 auf. Die Rückwand 24 und die Seitenwände 26-1, 26-2 sind in der Fig. 2 im Wesentlichen vertikal ausgerichtet.

Die Haltestruktur 22 ist dazu ausgebildet, eine Mehrzahl von Behältern 28 zur Aufnahme der Insekten zu halten. Die Behälter 28 erstrecken sich zwischen den Seitenwänden 26-1 und 26-2 der Haltestruktur 22. Beispielsweise können die Behälter 28 an den Seitenwänden 26-1, 26-2 und/oder an der Rückwand 24 gehalten sein. In der dargestellten Ausführungsform sind exemplarisch die Seitenwände 26-1, 26-2 an der Hängeeinrichtung 12 um die horizontale Schwenkachse 18 schwenkbar gelagert. Dies ist jedoch nicht zwingend. Stattdessen können auch andere Komponenten der Haltestruktur 22 drehbar an Hängeeinrichtung 12 gelagert sein. Die Haltestruktur 22 ist vorzugsweise zumindest teilweise formstabil ausgebildet. Beispielsweise können die Seitenwände 26-1, 26-2 formstabil ausgebildet sein.

Jeder Behälter 28 bildet eine Kammer 20 zur Aufnahme der Insekten aus. In Fig. 2 sind exemplarisch zwei Behälter 28 dargestellt. Es versteht sich jedoch, dass mehr als zwei Behälter 28 an der Haltestruktur 22 gehalten sein können. Die Behälter 28 sind in der ersten Stellung, insbesondere in einer Höhenrichtung, übereinander angeordnet.

Jeder Behälter 28 weist einen Behälterboden 30 und eine den Behälterboden 30 vollständig umgebende und von dem Behälterboden 30 aufragende Behälterseitenwand 32 auf. Die Behälterböden 30 sind in der in Fig. 2 gezeigten Darstellung im Wesentlichen horizontal ausgerichtet.

Jede Behälterseitenwand 32 weist einander gegenüberliegende Behälterseitenlängswände 32-1 und 32-2 auf, die Behälterseitenquerwände 32-3 und 32-4 miteinander verbinden. Die Behälterseitenlängswände 32-1, 32-2 erstrecken sich hierbei vorzugsweise im Wesentlichen parallel zu der horizontalen Schwenkachse 18.

Zur Oberseite hin sind die Behälter 28 jeweils offen ausgebildet, was bspw. für eine bessere Belüftung der Insekten sorgt. Optional kann jeder Behälter 28 einen nicht dargestellten Deckel zum, insbesondere lösbaren, Verschließen der Behälter 28 aufweisen.

Die Haltestruktur 22 kann optional eine Deckwand 34 und/oder eine Bodenwand 36 zum Verbinden der Seitenwände 26-1 und 26-2 der Haltestruktur 22 aufweisen, um die Stabilität der Haltestruktur 22 zu erhöhen. Eine Vorderseite 35 der Haltestruktur 22 kann geöffnet verschließbar ausgebildet sein.

Die Behälter 28 sind vorzugsweise derart an der Haltestruktur 22 gehalten, dass sie die Rückwand 24 kontaktieren. Die Rückwand 24 ist vorzugsweise als Gewebe, insbesondere als Gaze, ausgebildet, das die übereinander angeordneten Behälter 28 überspannt. Zu diesem Zweck kann das Gewebe lösbar mit der Haltestruktur 22 (z.B. mit den Seitenwänden 26-1, 26-2) gekoppelt sein. Beispielsweise kann das Gewebe durch miteinander verbundene Streben (nicht dargestellt) aufgespannt sein, die lösbar mit der Haltestruktur 22 koppelbar sind. Ein solches Gewebe kann optional auch an einer Vorderseite 35 der Haltestruktur 22 angeordnet sein. Die Behälter 28 können jedoch auch Teil der Rückwand 24 sein bzw. einstückig mit dieser ausgebildet sein. Vorzugsweise ist die Rückwand 24 in diesem Fall zumindest teilweise steif bzw. starr ausgebildet.

Das lösbar mit der Haltestruktur 22 gekoppelte Gewebe kann beispielsweise bei einem Entladen der Kammern 20 von Vorteil sein. Beispielsweise kann die Aufnahmeeinrichtung 16 derart in die zweite Stellung verschwenkt werden, dass die als Gewebe ausgebildete Rückwand 24 nach unten weist und einen Tragboden zum Tragen der Insekten bildet. Durch Entfernen des Gewebes in der zweiten Stellung, insbesondere in der Horizontalstellung, können die Insekten unter Einfluss der Schwerkraft über die in der zweiten Stellung nun geöffnete Rückwand 24 aus den Kammern 20 abgeführt bzw. entfernt werden. Das voranstehend beschriebene Entladeprinzip ist nicht auf eine Rückwand 24 beschränkt, die als Gewebe ausgebildet ist. Vielmehr kommt auch eine starre Rückwand 24 in Betracht, die lösbar mit der Haltestruktur 22 (z.B. mit den Seitenwänden 26-1, 26-2) gekoppelt ist.

Die Wahl der Porengröße des Gewebes hängt von der Größe der zu transportierenden Insekten ab. Im Allgemeinen sollten die Poren des Gewebes klein genug sein, um zu verhindern, dass die Insekten durch das Gewebe fallen. Vorzugsweise beträgt die (durchschnittliche) Porengröße des Gewebes 0,05 bis 6 mm, insbesondere 0,1 bis 5 mm.

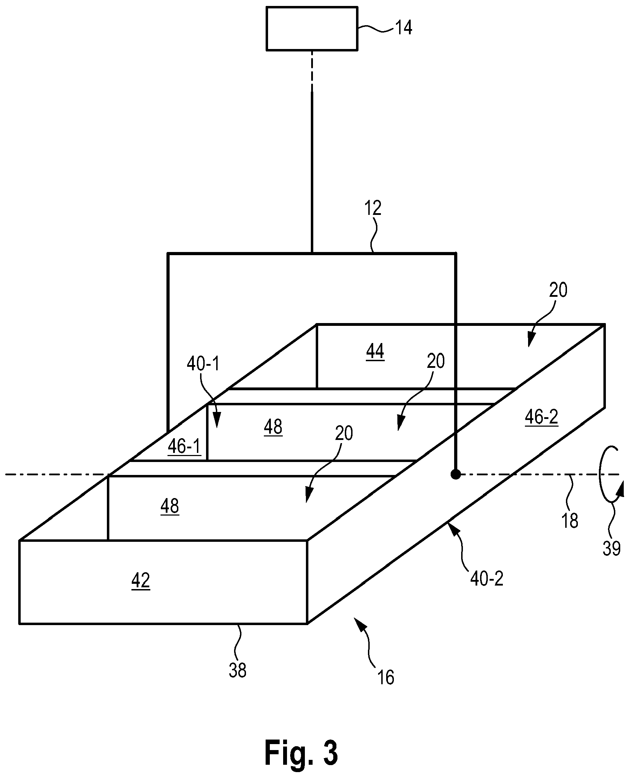

Fig. 3 zeigt eine isometrische Ansicht einer bevorzugten alternativen Ausführungsform der Hängeförder-Aufzuchtvorrichtung 10 aus Fig. 1A. Gleiche Elemente sind mit gleichen Bezugszeichen gekennzeichnet und werden nicht näher erläutert.

In der in Fig. 3 dargestellten Ausführungsform der Hängeförder-Aufzuchtvorrichtung 10 befindet sich die Aufnahmeeinrichtung 16 exemplarisch in der zweiten Stellung.

Die Aufnahmeeinrichtung 16 weist einen Rahmen 38 mit Stirnseiten 40-1, 40-2, einer Bodenwand 42, einer Deckwand 44, einander gegenüberliegenden Seitenwänden 46-1, 46-2 und Trennwänden 48, die sich zwischen den Seitenwänden 46-1, 46-2 erstrecken und einen Innenraum des Rahmens 38 in die Kammern 20 unterteilen, auf. In der gezeigten Darstellung weist der Rahmen 38 exemplarisch zwei Trennwände 48 auf, die den Innenraum des Rahmens 38 in drei Kammern 20 unterteilen.

In der ersten Stellung, die durch Verschwenken der Aufnahmeeinrichtung 16 um 90 Grad in der durch den Pfeil 39 angezeigten Richtung eingenommen wird, ist die untere der exemplarisch dargestellten drei Kammern 20 durch die Bodenwand 42, die untere der beiden Trennwände 48 sowie die Seitenwände 46-1, 46-2 gebildet. Ferner ist in der ersten Stellung die mittlere der drei Kammern 20 durch die beiden Trennwände 48 und die Seitenwände 46-1, 46-2 gebildet. Schließlich ist in der ersten Stellung die obere der drei Kammern 20 durch die obere der beiden Trennwände 48, die Deckwand 44 und die Seitenwände 46-1, 46-2 gebildet.

Die Kammern 20 erstrecken sich zwischen den Stirnseiten 40-1 und 40-2 des Rahmens 38. Vorzugsweise sind die Kammern 20 zu zumindest einer der beiden Stirnseiten 40-1 und 40-2 hin geschlossen oder verschließbar ausgebildet. Beispielsweise kann zumindest eine der Stirnseiten 40-1, 40-2 des Rahmens 38 als Wandung ausgebildet sein, die die Kammern 20 stirnseitig verschließt. Die Wandung weist vorzugsweise eine Vielzahl von nicht dargestellten Öffnungen auf. Beispielsweise kann die Wandung gelocht, siebförmig oder perforiert ausgebildet sein. Alternativ kann die Wandung netzartig, gitterartig oder gewebeartig ausgebildet sein. Beispielsweise kann zumindest eine der Stirnseiten 40-1, 40-2 des Rahmens 38 mit einem Gewebe, insbesondere einer Gaze, bespannt sein, das die Kammern 20 stirnseitig verschließt. In der in Fig. 3 dargestellten Ausführungsform sind beispielhaft die Seitenwände 46-1, 46-2 des Rahmens 38 drehbar an der Hängeeinrichtung 12 gelagert.

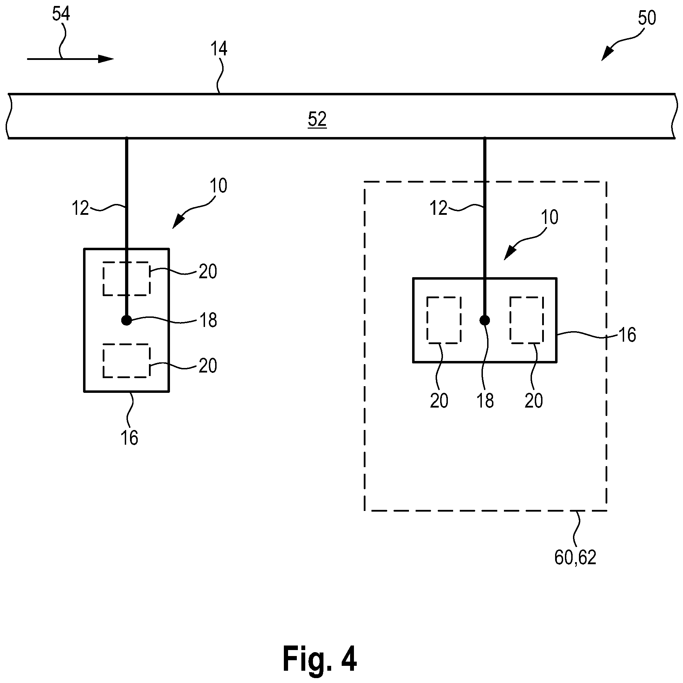

Fig. 4 zeigt eine schematische Darstellung einer Ausführungsform einer Hängeförderanlage 50 gemäß einem zweiten Aspekt der Erfindung. Gleiche Elemente sind mit gleichen Bezugszeichen gekennzeichnet und werden nicht näher erläutert.

Die Hängeförderanlage 50 weist einen Hängeförderer 14 mit einer Führungsschiene 52 auf, entlang der vorzugsweise ein nicht dargestelltes Zugmittel (z.B. eine Kette) bewegt und geführt wird, das wiederum mit der Hängeförder-Aufzuchtvorrichtungen 10 in Verbindung steht, um die Hängeförder-Aufzuchtvorrichtungen 10 entlang der Führungsschiene 52 durch die Anlage 50 zu bewegen. Die Hängeförder-Aufzuchtvorrichtungen 10 werden in einer Förderrichtung 54 entlang der Führungsschiene 52 bewegt. Die Förderrichtung 54 ist parallel zur Führungsschiene 52 orientiert und folgt dem Verlauf der Führungsschiene 52 in allen drei Raumrichtungen.

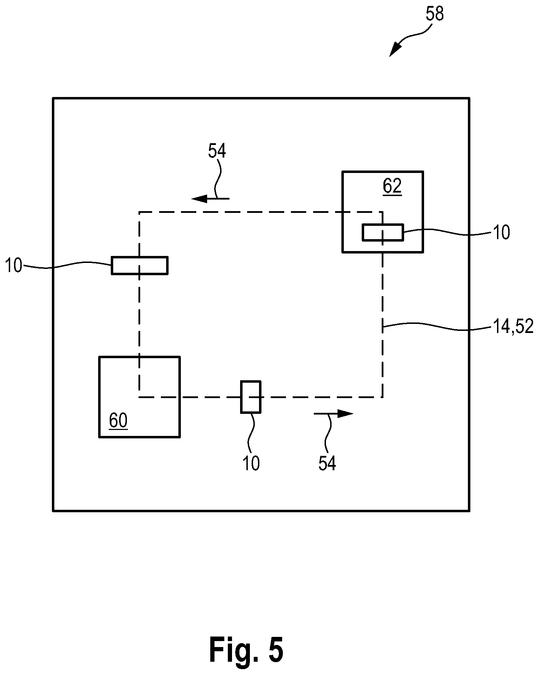

Die Aufnahmeeinrichtungen 16 der an den Hängeförderer 14 gekoppelten Hängeförder-Aufzuchtvorrichtungen 10 befinden sich jeweils in unterschiedlichen Stellungen. Die Aufnahmeeinrichtung 16 der in Bezug auf die Förderrichtung 54 stromaufwärts gelegenen Hängeförder-Aufzuchtvorrichtung 10 befindet sich in der ersten Stellung. Die Aufnahmeeinrichtung 16 der in Bezug auf die Förderrichtung 54 stromabwärts gelegenen Hängeförder-Aufzuchtvorrichtung 10 befindet sich in der zweiten Stellung, in der die Insekten verarbeitet werden. Die Verarbeitung der Insekten kann beispielsweise in einer Verarbeitungsstation 60, 62 einer nachfolgend in Fig. 5 näher erläuterten Insektenaufzuchtfarm 58 erfolgen.

Fig. 5 zeigt eine schematische Darstellung einer Insektenaufzuchtfarm 58 gemäß einem dritten Aspekt der Erfindung. Gleiche Elemente sind mit gleichen Bezugszeichen gekennzeichnet und werden nicht näher erläutert.

Die Insektenaufzuchtfarm 58 weist eine erste Verarbeitungsstation 60 und eine zweite Verarbeitungsstation 62 auf. Des Weiteren weist die Insektenaufzuchtfarm 58 eine Hängeförderanlage 50 mit einem Hängeförderer 14 und einer Führungsschiene 52 auf, entlang der vorzugsweise ein nicht dargestelltes Zugmittel (z.B. eine Kette) bewegt und geführt wird, das wiederum mit den Hängeförder-Aufzuchtvorrichtungen 10 in Verbindung steht, um die Hängeförder-Aufzuchtvorrichtungen 10 entlang der Führungsschiene 52 in einer Förderrichtung 54 zwischen den Verarbeitungsstationen 60 und 62 zu bewegen. Die Aufnahmeeinrichtungen 16 der Hängeförder-Aufzuchtvorrichtungen 10 können, wie bereits im Zusammenhang mit Fig. 4 erläutert, verschiedene Stellungen annehmen. Vorzugsweise befinden sich die Aufnahmeeinrichtungen 16 der Hängeförder-Aufzuchtvorrichtungen 10 während einer fortgesetzten Bewegung entlang der Führungsschiene 52 in der ersten Stellung. In den Verarbeitungsstationen 60 und 62 werden die Aufnahmeeinrichtungen 16 dann vorzugsweise in die zweite Stellung bewegt, um die Insekten zu verarbeiten. Es versteht sich, dass die Hängeförderanlage 50 auch mehrere Führungsschienen 52 zum Transportieren und/oder Lagern der Insekten aufweisen kann.

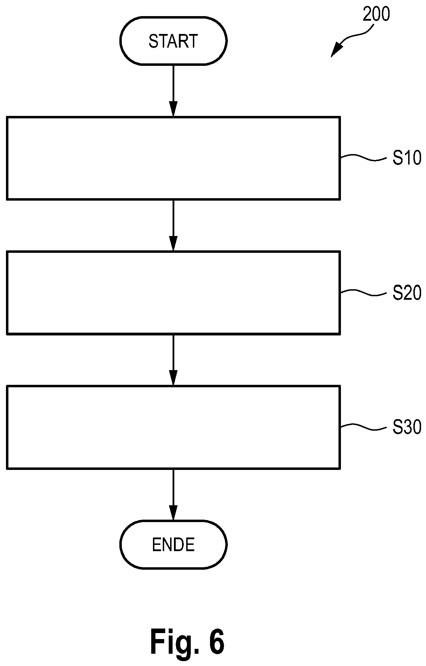

Fig. 6 zeigt ein Flussdiagramm eines Verfahrens zum Betreiben einer Hängeförder-Aufzuchtvorrichtung gemäß einem ersten Aspekt der Erfindung.

In einem ersten Schritt S10 wird zumindest eine an einen Hängeförderer 14 einer Hängeförderanlage 50 gekoppelte Hängeförder-Aufzuchtvorrichtung 10 gemäß dem ersten Aspekt in einer Verarbeitungsstation 60, 62 einer Insektenaufzuchtfarm 58 bereitgestellt.

In einem zweiten Schritt S20 wird die Aufnahmeeinrichtung 16 der zumindest einen Hängeförder-Aufzuchtvorrichtung 10 zwischen der ersten Stellung und der zweiten Stellung verschwenkt. Das Verschwenken der Aufnahmeeinrichtung 16 kann manuell oder automatisiert erfolgen.

In einem dritten Schritt S30 werden die Insekten in der Verarbeitungsstation 60, 62 der Insektenaufzuchtfarm 58 verarbeitet, während die Aufnahmeeinrichtung 16 sich in der ersten Stellung oder in der zweiten Stellung befindet. Vorzugsweise wird die Aufnahmeeinrichtung 16 in der Verarbeitungsstation 60, 62 von der ersten Stellung in die zweite Stellung bewegt, um die Insekten in der zweiten Stellung der Aufnahmeeinrichtung 16 zu verarbeiten.

## Patentansprüche

1. Hängeförder-Aufzuchtvorrichtung (10), aufweisend:
- eine Hängeeinrichtung (12) zur Kopplung an einen Hängeförderer (14); und
- zumindest eine Aufnahmeeinrichtung (16), die an der Hängeeinrichtung (12) um eine horizontale erste Schwenkachse (18) schwenkbar gelagert ist, wobei die Aufnahmeeinrichtung (16) zwischen einer ersten Stellung, in der die Aufnahmeeinrichtung (16) im Wesentlichen vertikal ausgerichtet ist, und einer von der ersten Stellung verschiedenen zweiten Stellung um die erste Schwenkachse (18) verschwenkbar ist und eine Mehrzahl von in der ersten Stellung übereinander angeordneten Kammern (20) zur Aufnahme von Insekten aufweist.

2. Hängeförder-Aufzuchtvorrichtung (10) nach Anspruch 1, wobei die zumindest eine Aufnahmeeinrichtung (16) in der zweiten Stellung im Wesentlichen horizontal ausgerichtet ist.

3. Hängeförder-Aufzuchtvorrichtung (10) nach Anspruch 1 oder 2, wobei die Aufnahmeeinrichtung (16) einen Rahmen (38) mit Stirnseiten (40-1, 40-2), einer Bodenwand (42), einer Deckwand (44), einander gegenüberliegenden Seitenwänden (46-1, 46-2) und zumindest einer Trennwand (48), die sich zwischen den Seitenwänden (46-1, 46-2) erstreckt und einen Innenraum des Rahmens (38) in die Mehrzahl von Kammern (20) unterteilt, aufweist.

4. Hängeförder-Aufzuchtvorrichtung (10) nach Anspruch 3, wobei die Kammern (20) sich zwischen den Stirnseiten (40-1, 40-2) des Rahmens (38) erstrecken.

5. Hängeförder-Aufzuchtvorrichtung (10) nach Anspruch 3 oder 4, wobei die Kammern (20) zu zumindest einer der beiden Stirnseiten (40-1, 40-2) hin geschlossen oder verschließbar sind.

6. Hängeförder-Aufzuchtvorrichtung (10) nach einem der Ansprüche 3 bis 5, wobei zumindest eine der Stirnseiten (40-1, 40-2) des Rahmens (38) als Wandung ausgebildet ist, wobei die Wandung die Mehrzahl von Kammern (20) stirnseitig verschließt, insbesondere wobei die Wandung gelocht, siebförmig oder perforiert oder netzartig ausgebildet ist.

7. Hängeförder-Aufzuchtvorrichtung (10) nach einem der Ansprüche 1 oder 2, wobei die zumindest eine Aufnahmeeinrichtung (16) eine Mehrzahl von Behältern (28) zur Aufnahme der Insekten und eine, insbesondere zumindest teilweise formstabile, Haltestruktur (22) zum Halten der Mehrzahl von Behältern (28) aufweist, wobei die Behälter (28) in der ersten Stellung übereinander angeordnet sind, und wobei jeder Behälter (28) der Mehrzahl von Behältern (28) eine Kammer (20) der Mehrzahl von Kammern (20) ausbildet.

8. Hängeförder-Aufzuchtvorrichtung (10) nach Anspruch 7, wobei jeder Behälter (28) einen Behälterboden (30) und eine den Behälterboden (30) zumindest teilweise, insbesondere vollständig, umgebende und von dem Behälterboden (30) aufragende Behälterseitenwand (32) aufweist, insbesondere wobei jeder Behälterboden (30) in der ersten Stellung im Wesentlichen horizontal ausgerichtet ist.

9. Hängeförder-Aufzuchtvorrichtung (10) nach einem der Ansprüche 7 oder 8, wobei die Haltestruktur (22) eine Rückwand (24) und zwei von der Rückwand (24) nach vorn abstehende, insbesondere formstabile, Seitenwände (26-1, 26-2), zwischen denen die Mehrzahl von Behältern (28) gehalten ist, aufweist, wobei die sich zwischen den Seitenwänden (26-1, 26-2) erstreckenden Behälter (28) die Rückwand (24) kontaktieren, insbesondere wobei die Seitenwände (26-1, 26-2) an der Hängeeinrichtung (12) um die horizontale erste Schwenkachse (18) schwenkbar gelagert sind.

10. Hängeförder-Aufzuchtvorrichtung (10) nach Anspruch 9, wobei die Rückwand (24) als Gewebe, insbesondere als Gaze, ausgebildet ist, das die Behälter (28) überspannt.

11. Hängeförder-Aufzuchtvorrichtung (10) nach einem der Ansprüche Anspruch 7 oder 8, wobei jeder Behälter (28) drehbar um eine zweite Schwenkachse an der Haltestruktur (22) gelagert ist.

12. Hängeförder-Aufzuchtvorrichtung (10) nach Anspruch 11, wobei die zweite Schwenkachse parallel zu der ersten Schwenkachse (18) verläuft, insbesondere wobei der zumindest eine Behälter (28) derart frei drehbar an der Haltestruktur (22) gelagert ist, dass der zumindest eine Behälter zumindest in der ersten Stellung und in der zweiten Stellung, insbesondere zwischen der ersten Stellung und der zweiten Stellung, orientierungsstabil ist.

13. Hängeförderanlage (50) zur Beförderung von Insekten, mit:
- zumindest einer Hängeförder-Aufzuchtvorrichtung (10) nach einem der Ansprüche 1 bis 12; und
- einem Hängeförderer (14) zur Beförderung der zumindest einen Hängeförder-Aufzuchtvorrichtung (10) entlang einer Führungsschiene (24) stromabwärts in einer Förderrichtung (54).

14. Insektenaufzuchtfarm (58) für die Aufzucht von Insekten, aufweisend:
- zumindest eine Verarbeitungsstation (60, 62) zur Verarbeitung der Insekten; und
- eine Hängeförderanlage (50) mit zumindest einer Hängeförderer-Aufzuchtvorrichtung (10) nach einem der Ansprüche 1 bis 12 und einem Hängeförderer (14) zur Beförderung der zumindest einen Hängeförderer-Aufzuchtvorrichtung entlang einer Führungsschiene (52) zu der zumindest einen Verarbeitungsstation (60, 62) und/oder von der zumindest einen Verarbeitungsstation weg (60, 62).

15. Verfahren (200) zum Betreiben einer Hängeförder-Aufzuchtvorrichtung (10) nach einem der Ansprüche 1 bis 12, mit den folgenden Schritten:
- Bereitstellen (S10) der zumindest einen an einen Hängeförderer einer Hängeförderanlage gekoppelten Hängeförder-Aufzuchtvorrichtung in einer Verarbeitungsstation einer Insektenaufzuchtfarm;
- Schwenken (S20) der Aufnahmeeinrichtung zwischen der ersten Stellung und der zweiten Stellung; und
- Verarbeiten (S30) der Insekten in der Verarbeitungsstation, während die Aufnahmeeinrichtung sich in der ersten Stellung oder in der zweiten Stellung befindet.
